# EUROPÄISCHE PATENTANMELDUNG

(11) **EP 2 050 389 A1**
(43) Veröffentlichungstag der Anmeldung: **22.04.2009**
(21) Anmeldenummer: 07020409.4
(22) Anmeldetag: 18.10.2007
(51) Int. Cl.: A61B 5/0496

(54) **Analyse-Einrichtung und Verfahren zur Bestimmung der Augenbewegungen**

(71) Anmelder: ETH Zürich, 8092 Zürich (CH)
(72) Erfinder: Bulling, Andreas, 8057 Zürich (CH)
(74) Vertreter: Kley, Hansjörg

(57) **Zusammenfassung**

Eine Analyse-Einrichtung (20) und ein Verfahren zur Bestimmung der Augenbewegungen ermöglichen, mittels dem EOG-Verfahren erfasste Signale unabhängig von externen Einflüsse zu erfassen. Dazu werden mittels Elektroden (2) EOG-Signale aufgezeichnet und in Echtzeit direkt auf einer Analyse-Einheit (10) ausgewertet. Die Umgebungseinflüsse Licht, Bewegung und Feuchtigkeit werden mit einem Lichtsensor (4) bzw. einem Beschleunigungssensor (4) bzw. einem Feuchtigkeitssensor (4) erfasst. Dies ermöglicht die adaptive Anpassung an sich verändernde Umgebungen während der Dauer von Langzeitaufnahmen. Die Adaptivität erstreckt sich dabei explizit bis auf die Software-Schicht, wo abhängig von den Betriebsparametern vom System selbständig die jeweiligen Algorithmen ausgewählt und die notwendigen Signalverarbeitungsschritte durchgeführt werden.

## Beschreibung

Die vorliegende Erfindung bezieht sich auf eine Analyse-Einrichtung und Verfahren zur Bestimmung der Augenbewegungen nach dem Oberbegriff der Patentansprüche 1 und 6.

Zur Untersuchung der Augenbeweglichkeit und zur Diagnose von Störungen des Gleichgewichtssystems müssen Patienten eine spezialisierte Klinik oder einen spezialisierten Arzt aufsuchen. Während einer solchen Konsultation von etwa 5 bis 20 Minuten Dauer werden die Augenbewegungen aufgezeichnet und ausgewertet. Wünschbar ist, dass die Augenbewegungen einer Person über einen längeren Zeitraum und unabhängig vom Einfluss einer speziell dafür vorgesehenen Untersuchung durchgeführt werden können.

Für die Erfassung der Blickrichtung existieren Lösungen, die Daten einerseits zur Steuerung einer Mensch-Computer-Interaktion wie auch ggf. für eine medizinische Analyse liefern.

In WO 2002/065898 A2 bzw. EP 1 405 157 B1 [1] ist eine Vorrichtung und ein Verfahren zur Blickrichtungsbestimmung bezüglich eines festen Referenzkoordinatensystems offenbart. Dieser in [1] offenbarte Gegenstand benutzt folgende Gegebenheit: Im Auge besteht ein ständiger, elektrischer Spannungsunterschied zwischen Vorder- und Rückseite der Netzhaut, der dazu führt, dass die Retina negativ und die Rückseite des Augapfels (Kornea) positiv geladen sind (Dipol). Dieser Unterschied wird auch als Ruhepotential bezeichnet. Durch Augenbewegungen nähert sich die Vorderseite des Auges der einen Elektrode an, während die Rückseite sich der anderen Elektrode annähert. Dadurch kommt es zu einer Spannungsdifferenz, die nach einer Signalverstärkung zwischen zwei Elektroden gemessen werden kann, die links und rechts (beziehungsweise oberhalb und unterhalb) des Auges auf der Haut angebracht sind. Diese Aufzeichnung wird als Elektrookulographie (EOG) bezeichnet, das Signal selbst wird Elektrookulogramm genannt. Nimmt man an, dass das Ruhepotential der Netzhaut konstant ist, so ist diese Spannungsdifferenz ungefähr proportional zum Blickwinkel und ihre Messung ermöglicht damit die Rekonstruktion der Augenbewegungen. Da Augenbewegungen nicht nur willkürlich, sondern auch über das Gleichgewichtssystem gesteuert werden, wird das Verfahren nicht nur zur Diagnose von Störungen des Augenbewegungs- sondern auch des Gleichgewichtssystems eingesetzt. Bei der Schlafpolygraphie dient die EOG der Erkennung und Analyse der REM-Phasen.

Gemäss [1] sind auf einem Brillengestell Elektroden angebracht, so dass diese Elektroden beim Tragen durch eine Person in der Augenumgebung in Kontakt mit der Haut sind. Gemäss dem in [1] aufgeführten, umfangreichen Stand der Technik wurde eine Vorrichtung und ein Verfahren geschaffen, das auf der Basis eines Elektrookulogramms eine für einen Benutzer bequeme Schnittstelle schafft, damit die Blickrichtung der Augen bezüglich eines festen Referenzkoordinatensystems erfasst und damit z.B. ein Cursor auf einem Bildschirm bewegt werden kann.

Die Erfassung der Augenbewegung gemäss dem in [1] und im Stand der Technik bekannten Verfahren ist jedoch nicht resistent gegenüber folgenden Einflussgrössen bzw. Umgebungseinflüssen:
- Umgebungslicht bzw. allgemein Beleuchtungssituation;
- Kopfbewegungen, insbesondere unfreiwillige oder schnelle Kopfbewegungen;
- Körperaktivität wie Laufen, Springen, etc.;
- Kopf-Augen-Interaktion bzw. optokinetische Effekte bei gleichzeitiger Bewegung von Kopf und Augen;
- Hautfeuchtigkeit und damit Widerstand zwischen Haut und Elektroden.

Die Lösung gemäss [1] erfordert zudem eine dauernde galvanische Kopplung mit einem Rechner.

In dieser Schrift wird anstelle des Begriffs Analyse-Einrichtung zur Bestimmung der Augenbewegungen bzw. «Vorrichtung» wie in [1] genannt der Kürze und Prägnanz zuliebe von nun an der synonyme Begriff «Eye-Tracker» verwendet. Entsprechend wird für ein Verfahren zur Bestimmung der Augenbewegungen der synonyme Begriff «Verfahren zum Eye-Tracking» oder auch schlicht «Eye-Tracking» verwendet.

Der vorliegenden Erfindung liegt die Aufgabe zugrunde einen Eye-Tracker und ein Verfahren zum Eye-Tracking anzugeben, das gegenüber externen Einflüsse auf das gemäss dem Stand der Technik praktizierte Eye-Tracking resistent ist bzw. diese Einflüsse in Echtzeit automatisch kompensiert und einen Langzeiteinsatz auch ausserhalb der klinischen Umgebung ermöglicht.

Diese Aufgabe wird durch die in den Patentsprüchen 1 und 6 angegebenen Merkmale gelöst.
Die vorliegende Erfindung erlaubt somit mit den von einem oder mehreren Sensoren erfassten Signalen eine Kompensation und adaptive Anpassung der EOG-Signalverarbeitung/-Auswertung in Echtzeit.

Besondere Ausführungsformen zeichnen sich durch folgende Eigenschaften aus:
- System zur Aufzeichnung von Elektrookulogrammen in einem tragbaren und über einen langen Zeitraum einsetzbaren Aufbau, bezüglich der Signalaufzeichnung vergleichbar zu bereits verfügbaren EKG Lösungen; ermöglicht durch integriertes, leistungsfähiges low-power/low-weight System mit spezieller Analogverstärkerschaltung, Speichervorrichtung und kabellose Datenübertragung (z.B. für klinische Diagnostik, Erkennung/Voraussage bestimmter psychischer Krankheiten);
- Berechnung der Augenbewegungen aus EOG Signalen zu einem Bezugssystem wie Kopf oder Umgebung;
- Langzeit-Aufnahme von Augenbewegungen (im Gegensatz zu videobasierten Lösungen, die gegenwärtig auf ca. max. 2h Aufnahmedauer beschränkt sind) ermöglicht z.B. Trend-Analyse. Dies umgeht gleichzeitig auch das Baseline-Drift Problem, da nur längerfristige Änderungen im EOG-Signal betrachtet werden müssen;
- Aufnahmen der Augenbewegungen während eines Tages; ermöglicht z.B. die Analyse von impliziten/kognitiven Aspekten der visuellen Wahrnehmung, wie Ablenkung (z.B. im Strassenverkehr), Konzentration oder Intention; ermöglicht vor allem durch Unaufdringlichkeit des Systems, welches das natürliche Verhalten nicht beeinflusst und damit aussagekräftige Ergebnisse sicherstellt;
- Integration von EOG-Messungen in Body-Sensor-Netzwerken oder Krankenhaussystemen zur physiologischen Überwachung, ermöglicht durch kabellose Übertragung und Echtzeitbetriebssystem.

Vorteilhafte Weiterbildungen sind Gegenstand der abhängigen Ansprüche.

Ausführungsbeispiele der vorliegenden Erfindung werden nachstehend anhand der Figuren erläutert:
- Fig. 1: Prinzipschema der Sensoren auf dem Brillengestell und der Komponenten der Analyseeinheit;
- Fig. 2: Verfahrensschritte zur adaptiven Analyse des EOG-Rohsignals im Detail;
- Fig. 3: Verfahrensschritte zur Analyse des EOG-Rohsignals;
- Fig. 4: Komponenten der Analyse-Einrichtung umfassend Brillengestell und abgesetzte Analyse-Einheit;
- Fig. 5: Darstellung eines das Lesen durch eine Person repräsentierenden Signals;
- Fig. 6: Brillengestell mit der Anordnung der Elektroden und eines Hardware-Moduls.

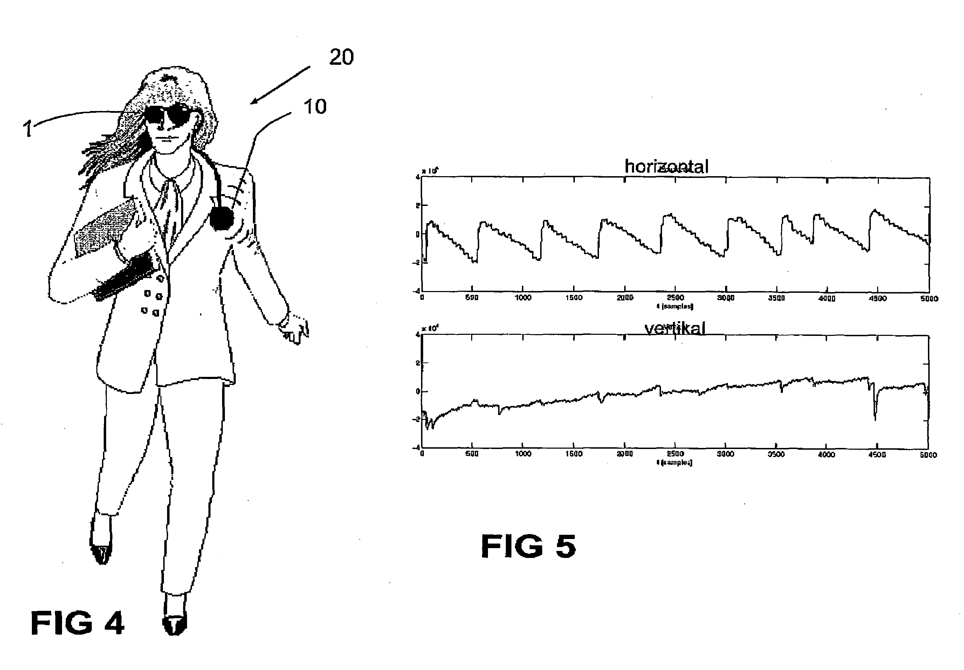

Die Figur 4 zeigt in einer ersten Ausführungsform beispielhaft die Komponenten der Analyse-Einrichtung 20 zur Bestimmung der Augenbewegungen, diese wird integral im folgenden kurz Eye-Tracker 20 genannt. Der Eye-Tracker 20 umfasst:
- ein Brillengestell 1 auf dem Elektroden 2 , ein Hardware-Modul 3, sowie ein Lichtsensor 4 angebracht sind und
- eine abgesetzte Analyse-Einheit 10.
In einer zweiten bevorzugten Ausführungsform ist die Analyse-Einheit 10 vollständig im Brillengestell 1 integriert.

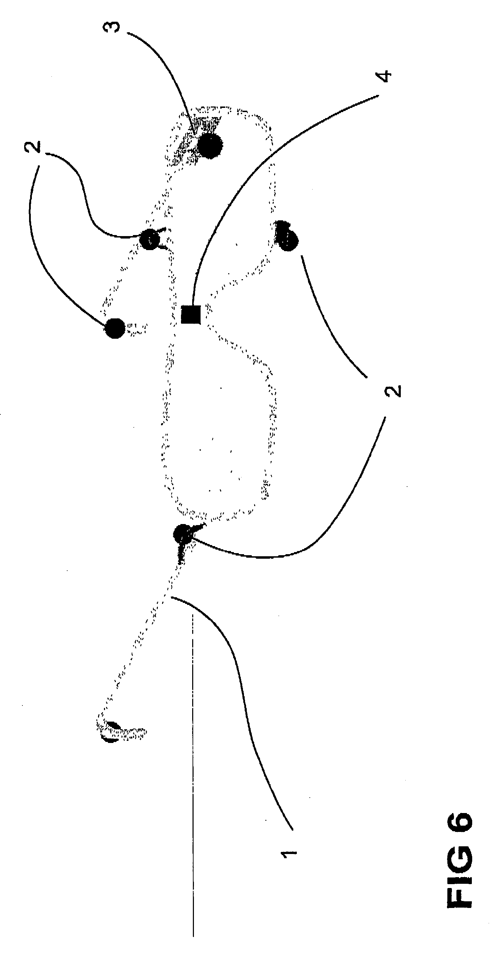

Für die Erläuterung der Signalaufnahme mit den Elektroden 2 wird auf die Figur 6 verwiesen. Das Brillengestell 1 weist die für das EOG-Aufnahmeverfahren notwendigen Elektroden 2 an den geeigneten Position auf. Zusätzlich sind noch weitere Sensoren 4 vorgesehen, um Umwelteinflüsse auf die Erfassung der EOG-Signale kompensieren zu können. Dazu ist gemäss Figur 6 beispielhaft ein nach vorne gerichteter Lichtsensor 4 am Brillengestell 1 angebracht. Dieser Lichtsensor 4 ermöglicht eine Kompensation, da das EOG-Messverfahren wie beschrieben auf einem Potential zwischen Kornea und Retina beruht, welches vom Umgebungslicht abhängig ist. Die Elektroden 2 sind kreisförmig um beide Augen angeordnet und mit am Brillengestell 1 befestigten bzw. in das Brillengestell 1 integrierten Kabeln mit dem Hardware-Modul 3 verbunden. Die Verbindung zwischen Lichtsensor 4 und Hardware-Modul 3 geschieht auf dieselbe Art und Weise.
Der Kontakt zwischen Elektroden 2 und Hautoberfläche wird auf mechanischem Weg durch kleine Blattfedern sichergestellt. Diese pressen die Elektroden 2 mit ungefähr gleichbleibendem Druck auf die Haut auf und vermindern so Bewegungsartefakte oder gar Unterbrechungen bei der Messung der Signale. Eine weitere Kompensation von Umgebungseinflüssen kann mit einem Beschleunigungssensor 4 vorgenommen werden (nicht dargestellt in Figur 6).

Das Hardware-Modul 3 ist direkt am Brillengestell 1 befestigt und enthält die spezielle EOG Biosignalverstärkerschaltung, einen Beschleunigungssensor 4 und Komponenten zur Anbindung und dem Auslesen des Lichtsensors 4. Die umgesetzte EOG-Schaltung lehnt sich stark an die bewährten Schaltungen für die Aufnahmen von EKG-Signalen an, wodurch insbesondere auch eine fünfte Elektrode 2 zur Definition des Referenzpotentials («Driven Right Leg») Verwendung findet. Gerade Einkopplungen des Stromnetzes können mit dieser Schaltung effektiv kompensiert werden. Das Hardware-Modul 3 ist gemäss der ersten Ausführungsform mit geschirmten Kabelverbindungen mit der Analyse-Einheit 10 verbunden. Diese geschirmten Kabelverbindungen verlaufen auf einer Seite hinter den Ohren über den Hals in Richtung Analyse-Einheit 10. Einfache Steckverbindungen ermöglichen, dass auch andere Komponenten bzw. Geräte zur Signalaufzeichnung bzw. analoge Verstärkerschaltungen benutzt werden könnten, sollte für eine bestimmte Anwendung beispielsweise eine Änderung der Art oder Anzahl der Elektroden 2 notwendig sein. Vorstellbar wäre z.B. die gleichzeitige Aufzeichnung der Bewegungen beider Augen oder die Verwendung feuchter Elektroden 2. Die standardmässig verwendeten, trockenen Elektroden 2 gewährleisten jedoch, dass das Brillengestell 1 mit diesen Sensoren 4 ohne Schwierigkeiten und schnell abgenommen und aufgesetzt werden kann.

Um die Kopfbewegungen messen zu können, die die Bewegungen der Augen stark beeinflussen, wird der Beschleunigungssensor 4 benötigt. Die Messwerte dieses Beschleunigungssensors 4, ebenso wie die erwähnten Lichtsensoren 4, können dann in Echtzeit in die Signalverarbeitung mit einfliessen, um eine Adaptivität auch bei sich verändernden Umgebungen und Betriebsbedingungen zu gewährleisten.

Eine zusätzliche Erweiterung der Analyse-Einheit 10 könnte in Form des Hinzufügens weiterer Sensoren 4, wie zum Beispiel Magnetometer oder Gyroskop, bestehen. Dies würde neben der Verfolgung und Aufzeichnung von Augenbewegungen gleichzeitig auch das Tracking des Kopfes ermöglichen und dem Eye-Tracker weitere Einsatzmöglichkeiten eröffnen. Als weiteren Sensor 4 kann ein Feuchtigkeitsmesser 4 für die Hautfeuchtigkeit vorgesehen werden, um eine Kompensation von Änderungen des Hautwiderstandes vorzunehmen.

Eine weitere Miniaturisierung und die direkte Integration der Analyse-Einheit 10 in das Brillengestell 1 gemäss der erwähnten zweiten Ausführungsform erlaubt die Aufzeichnung der Augenbewegungen in für die Person selbst und andere Personen vollkommen unsichtbarer Art und Weise.

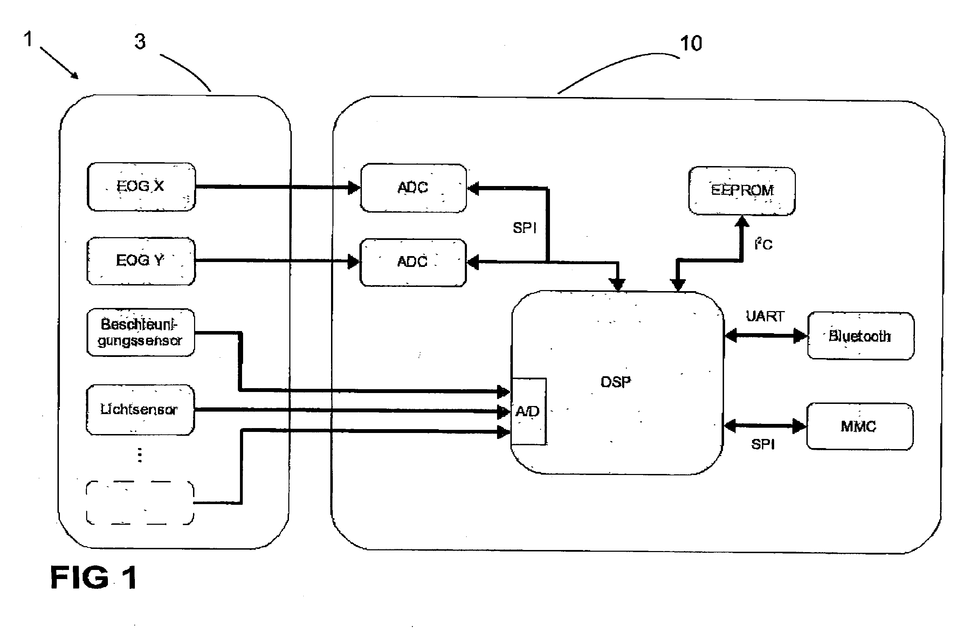

Auf der Analyse Einheit 10 zur Aufnahme von EOG-Signalen sind gemäss Fig. 1 aufgebracht: ein Mikrokontroller (z.B. DSP), eine Speicherkarte (z.B. MMC), ein Modul zur kabellosen Datenübertragung (z.B. Bluetooth), ein Lithium-Polymer-Akku mit Ladeschaltung und Komponenten zur AD-Wandlung. Diese Analyse Einheit 10 kann gemäss der ersten Ausführungsform zum Beispiel in der Brusttasche oder in einer Tasche am Arm getragen werden, um den Benutzer in seiner Bewegungsfreiheit nicht einzuschränken (vgl. Abbildung 4). Der digitale Signalprozessor DSP ermöglicht die Ausführung auch komplexer und rechenintensiver Signalverarbeitungsalgorithmen in Echtzeit. Diese können aufgrund des tragbaren Setups mit sich ständig verändernden Umgebungen und der Datenaufzeichnung unter Verwendung trockener Elektroden 2 notwendig werden. Der DSP erweitert jedoch auch die Anwendungsmöglichkeiten der Analyse-Einheit 10 insgesamt und erlaubt den flexiblen Austausch bzw. die Anpassung der Signalverarbeitungsalgorithmen zur Laufzeit. Die Verwendung eines Echtzeitbetriebssystems auf Softwareseite erlaubt zudem die Verwendung entsprechender (verteilter) Software-Architekturen (Middleware) und damit die Integration des der Analyse-Einheit 10 in andere (verteilte) Systemumgebungen zur Messung physiologischer Parameter. Die eingesetzten Analog-Digital-Konverter erlauben das Einstellen der Samplingrate und damit der Genauigkeit der Wandlung durch den DSP. Zur Durchführung von Langzeitaufnahmen über mehrere Stunden bzw. Tage ist eine Speicherkarte und eine entsprechende Schaltung integriert. Schon heute erlaubt eine Standard-Speicherkarte die Datenspeicherung von Aufnahmen über mehrere Tage bzw. Wochen erlauben. Das Bluetooth-Modul dient zum Einen der Datenübertragung nach oder während der Datenaufnahme, kann jedoch auch dazu benutzt werden, die Analyse-Einheit 10 in ein Body-Sensor-Netzwerk zu integrieren bzw. mit weiteren physiologischen Sensoren 4 zu vernetzen. Die Autonomie beträgt mit einem 1300 mAh Lithium-Polymer-Akku beim entwickelten Protoypen 7 Stunden.

Die für die Aufnahme der Augenbewegungen notwendige Funktionalität des Eye-Trackers 20 ist für den Benutzer sehr einfach mittels zweier Druckknöpfe bedienbar. Verschiedene LEDs geben während einer Aufnahme zudem ständig Rückmeldung über den aktuellen Betriebszustand und die Aktivität der Gerätes. Der Eye-Tracker 20 erlaubt jedoch gleichzeitig dem Entwickler bzw. der auswertenden Person der Daten umfangreiche Einstell- und Analysemöglichkeiten.

Nach dem Aufsetzen des Brillengestells 1 muss zunächst der Hautkontakt und die Position der Elektroden 2 überprüft werden. Es ist hierbei im Sinne einer guten Signalqualität von grosser Wichtigkeit, dass die Elektrodenpaare möglichst exakt auf den horizontalen und vertikalen Augenachsen positioniert werden. Eine ungenaue Positionierung ermöglicht zwar ebenso die EOG-Messung, die Qualität der Signale wird jedoch üblicherweise durch sogenannten Crosstalk (Messung von vertikalen Signalanteilen durch die horizontalen Elektroden 2 und umgekehrt) stark beeinträchtigt, was die Bestimmung der Augenbewegungen deutlich erschwert. Nach Aufsetzen des Brillengestells 1 muss dann die Kabelverbindung zur Analyse-Einheit hergestellt und diese eingeschaltet werden. Im Betriebsmodus mit kabelloser Datenübertragung muss als letzter Schritt zudem noch die kabellose Verbindung zum Aufnahmesystem (z.B. Laptop oder PDA) bzw. Wireless-Sensor-Netzwerk hergestellt werden, was auf Seiten des Eye-Trackers 20 nur das Drücken eines Knopfes erfordert.

Im Anschluss an die mechanische Kalibrierung kann zusätzlich noch an einem abgesetzten Computer eine softwaremässige Kalibrierung durchgeführt werden, um die Algorithmen zur Erkennung der Signalcharakteristiken initial auf die von,Person zu Person unterschiedlichen Signale anzupassen. Es ist gewünscht, diese Kalibrierung mobil und vom Eye-Tracker 20 selbst in einem interaktiven Ablauf z.B. über ein Headup-Display durchzuführen. Zur Kalibrierung wird der Benutzer gebeten, in einem definierten Abstand positionierte, von einer auf dem Computer installierten Softwareumgebung vorgegebenen Punkte der Reihe nach zu fixieren. Mit Hilfe dieser Fixationen können dann in der Softwareumgebung die Schwellwerte zur Erkennung von Sakkaden und Lidbewegungen gesetzt und validiert, sowie die allgemeine Signalqualität überprüft werden. Bei schlechter Signalqualität müssen ggf. die Positionierung der Elektroden 2 korrigiert werden. Die so gewonnen Parameter finden dann für die weiteren Aufnahmen Verwendung, müssen jedoch unter Umständen später von Zeit zu Zeit (entweder in Interaktion mit dem Benutzer oder automatisch im Hintergrund während der Aufnahme durch die auf dem Eye-Tracker ausgeführte Analyse-Software) neu bestimmt bzw. angepasst werden. Nach Abschluss der Kalibrierung ist der Eye-Tracker 20 auf diesen bestimmten Benutzer angepasst und es kann mit der mobilen Datenaufnahme begonnen werden.

Während der Aufnahme zeichnen die Elektroden 2 ständig die durch die Augenbewegungen hervorgerufenen Potentialveränderungen auf, die anderen Sensoren 4 die entsprechenden Beschleunigungs- und/oder Lichtsensorwerte. Die Daten werden in Echtzeit verarbeiten und je nach Betriebsmodus direkt weiter übertragen oder auf der integrierten Speicherkarte zwischengespeichert. Entsprechend demselben Betriebsmodus stehen nach Beendigung der Aufnahme die Daten dann entweder schon auf dem Aufnahmesystem oder noch auf der Speicherkarte zur Verfügung. Im ersten Fall hat die Aufnahmesoftware schon während der Aufzeichnung die binären Daten in ein menschenlesbares Format umgewandelt, im zweiten Fall übernimmt das eine Konvertierungsroutine der bereits mehrmals erwähnten Software-Umgebung.

Für den normalen Benutzer ist die Verwendung des Gerätes an dieser Stelle beendet, alle weiteren Schritte und Analysen können nun mit Hilfe der Softwareumgebung von einer auswertenden Person durchgeführt werden.

Der weiter oben beschriebene Schritt einer Kalibrierung kann hinsichtlich einer Benutzerinteraktion entfallen, wenn in der Analyse-Einheit 10 entsprechende Lernalgorithmen implementiert sind.

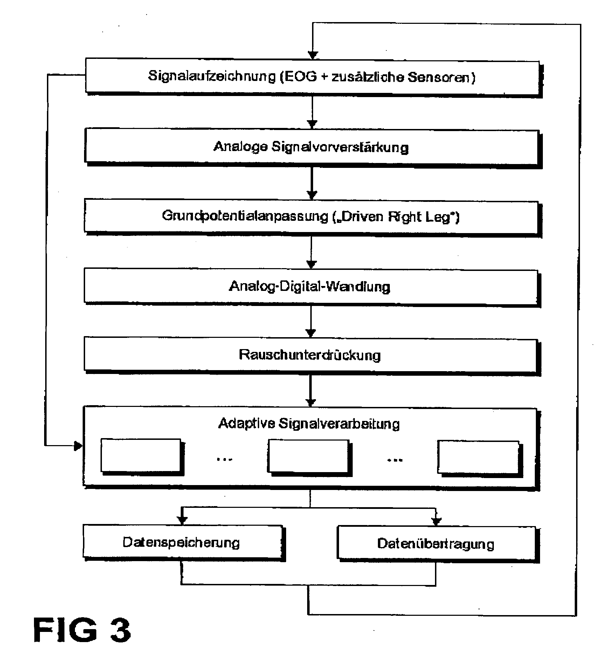

In dem in Fig. 3 dargestellten Diagramm ist der prinzipielle Ablauf der Aufnahme und Verarbeitung von EOG-Signalen auf dem Gerät dargestellt. Im ersten Schritt werden die EOG-Signale, sowie die Signale der anderen Sensoren 4 wie Beschleunigungssensor 4, Lichtsensor 4, etc. aufgezeichnet. Die analogen EOG-Signale werden daraufhin zunächst vorverstärkt und durchlaufen dann die bereits erwähnte Grundpotentialanpassung («Driven Right Leg»), die wie vergleichbare EKG-Verstärkerschaltungen aufgebaut ist. Nach der Analog-Digital-Wandlung auf dem Hauptmodul werden die Signale anschliessend durch Filterung von grundlegenden Störungen, wie z.B. Rauschen, befreit. An diese Rauschunterdrückung können sich dann eine variable Anzahl weiterer, adaptiver Signalverarbeitungsschritte anfügen, die sowohl vom Gerät selbst, als auch dem Benutzer ausgewählt und parametrisiert werden können. Nach Abschluss der Signalverarbeitung werden die Signale entweder der Datenspeicherung oder der kabellosen Datenübertragung zugeführt.

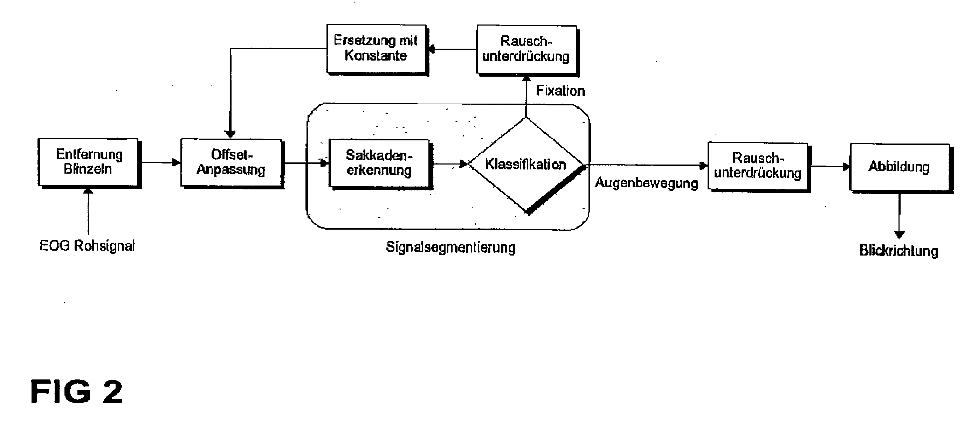

Ein beispielhafter Ablauf der im letzten Abschnitt erwähnten Signalverarbeitungsschritte, in diesem Fall mit dem Ziel der Berechnung der Blickrichtung, ist in Fig. 2 dargestellt. In der vertikalen Komponente des bereits von Rauschen befreiten EOG-Signals werden zunächst Lidbewegungen erkannt und abhängig von der Art der Bewegung entfernt. Nach einer Anpassung der Amplituden beider Komponenten (horizontal und vertikal) schliesst sich dann eine Signalsegmentierung an. Nach der Erkennung von Sakkaden werden dann Fixationen von anderen Augenbewegungen unterschieden. Nach einer erneuten Rauschunterdrückung wird das Signal dann entweder zur weiteren Verarbeitung zurückgeführt oder auf die Blickrichtung abgebildet.

Figur 5 zeigt beispielhaft den in horizontaler und vertikaler Richtung über die Zeit t erfassten und ausgewerteten Signalverlauf. In der oberen Darstellung ist eine Periodizität unverkennbar. Hier überstreicht ein Auge mit geringerer und einigermassen konstanter Geschwindigkeit einen bestimmte Länge. Dann erfolgt abrupt eine Rückkehr auf ungefähr den gleichen Ort, jedoch mit deutlich höherer Geschwindigkeit. In vertikaler Richtung ist für die gleichen Zeitabschnitte der bereits erwähnte Baseline-Drift erkennbar. Diese beiden Signalverläufe entsprechen der Augenbewegung beim Lesen eines Textes.

Zusammenfassend nochmals eine Erläuterung der verschiedenen Kompensation mittels von Sensoren 4 erfassten Signalen:
- - Kompensation von verschiedensten Arten von Bewegungsartefakten, die sich z.B. durch Bewegung des Kopfes, aber auch bei körperlicher Aktivität wie z.B. Laufen, etc. im gemessenen EOG-Signal niederschlagen können.
- - Kompensation von gleichzeitigen Bewegungen von Augen und Kopf, da eine gleichzeitige Kopfbewegung grossen Einfluss auf die Augenbewegung hat:
   So kompensiert der Kopf ab einem gewissen Winkelbereich einen Teil der Bewegung, so dass sich die Augen weniger/anders bewegen müssen. Dies wird mit einem Beschleunigungssensor 4 erfasst.
- - Kompensation von Änderungen im Umgebungslicht bzw. der Beleuchtungssituation, die das Ruhepotential und damit die von den Elektroden 2 gemessene Spannungsdifferenz beeinflussen. Dies wird mit einem Lichtsensor 4 erfasst.
- - Kompensation von Änderungen der Hautfeuchtigkeit und damit des Widerstandes zwischen Haut und Elektroden 2.

Vorstehend war stets von einem Brillengestell 1 die Rede. Für die vorliegende Erfindung ist es unerheblich, ob nun tatsächlich eine Brille oder nur ein Brillengestell 1 vorliegt, wichtig ist ein Träger für die Elektroden 2 und die zugehörigen Schaltungen, dafür genügt es, lediglich ein Brillengestell 1 vorzusehen.

### Liste der verwendeten Bezugszeichen; Glossar

- 1: Brillengestell
- 2: Elektroden; trockene Elektroden
- 3: Hardware-Modul
- 4: Sensor, Lichtsensor; Beschleunigungssensor; Feuchtigkeits-sensor
- 10: Analyse-Einheit, Hauptkomponente
- 20: Analyse-Einrichtung zur Bestimmung der Augenbewegungen, Eye-Tracker
- A/D: Analog/Digital
- ADC: Analog/Digital-Konverter
- DSP: Digital Signal Processor
- EEPROM: Electrically Erasable Programmable Read-Only Memory
- EKG: Elektrokardiogramm/-graphie
- EOG: Elektrookulogramm/-graphie
- GSR: Galvanic Skin Response
- I2C: Inter-Integrated Circuit
- MMC: Multimedia Card
- REM: Rapid Eye Movement
- Fixation: kurzzeitige, statische Augenpositionierung auf einen bestimmten Punkt
- Sakkade: schnelle Augenbewegungen zwischen zwei Fixationspunkten
- Driven: Right Leg Schaltung zur Reduktion von elektrischen Störungen in medizinischen Geräten
- Baseline-Drift: langsame Signalverschiebung unabhängig von tatsächlich stattfindenden Änderungen in der Signalquelle
- SPI: Serial Peripheral Interface
- UART: Universal Asynchronous Receiver/Transmitter

### Liste der zitierten Dokumente

[1] WO 2002/065898 A2
EP 1 405 157 B1
«Vorrichtung und ein Verfahren zur Blickrichtungsbestimmung bezüglich eines festen Referenzkoordinatensystems»
Fraunhofer-Gesellschaft zur Förderung der angewandten
Forschung e.V.
Hansastrasse 27c
80686 München / DE

## Patentansprüche

1. Analyse-Einrichtung (20) zur Bestimmung der Augenbewegungen einer Person, die enthält:
- Brillengestell (1), an dem Elektroden (2) angebracht sind, die mit der Haut in der Umgebung der Augen der Person in Kontakt sind, wobei am diese Elektroden (2) Signale für eine Elektrookulographie (EOG) zur Bestimmung der Augenbewegungen ableitbar sind;
- Analyse-Einheit (10), die aus den von den Elektroden (2) abgeleiteten Signalen die Augenbewegung bestimmt;
**dadurch gekennzeichnet, dass**
zur Kompensation von Umgebungseinflüssen auf die an den Elektroden (2) abgeleiteten Signale wenigstens ein Sensor (4) zur Aufnahme eines den Umgebungseinfluss repräsentierenden Signals vorgesehen ist und dass das von diesem Sensor (4) entstammende Signal der Analyse-Einheit (10) zuführbar ist und in der Analyse-Einheit (10) Mittel vorgesehen sind, um den Umgebungseinfluss in Echtzeit zu kompensieren.

2. Analyse-Einrichtung nach Anspruch 1,
**dadurch gekennzeichnet, dass**
als Sensor (4) auf dem Brillengestell (1) vorgesehen sind: ein Lichtsensor (4) und/oder ein Beschleunigungssensor (4) und/oder ein Feuchtigkeitssensor (4).

3. Analyse-Einrichtung nach Anspruch 1 oder 2,
**dadurch gekennzeichnet, dass**
als Elektroden (2) trockene Elektroden (2) vorgesehen sind.

4. Analyse-Einrichtung nach einem der Ansprüche 1 bis 3,
**dadurch gekennzeichnet, dass**
in der Auswerte-Einheit (10) zusätzlich vorgesehen sind:
- ein Speichermodul (MMC),um die kompensierten Signale zu speichern;
- ein Übertragungsmittel (Bluetooth), um die kompensierten Signale an ein externes Computersystem zu übermitteln.

5. Analyse-Einrichtung nach einem der Ansprüche 1 bis 4,
**dadurch gekennzeichnet, dass**
die Auswerte-Einheit (10) im Brillengestell (1) integriert ist.

6. Verfahren zur Bestimmung der Augenbewegungen einer Person, wobei
- mittels eines Brillengestells (1), an dem Elektroden (2) angebracht sind, die mit der Haut in der Umgebung der Augen der Person in Kontakt sind, werden von den Elektroden (2) Signale für eine Elektrookulographie (EOG) zum Zweck der Bestimmung der Augenbewegung erfasst;
**dadurch gekennzeichnet, dass**
zur Kompensation von Umgebungseinflüssen auf die an den Elektroden (2) abgeleiteten Signale wenigstens ein Sensor (4) zur Aufnahme eines den Umgebungseinfluss repräsentierenden Signals vorgesehen ist und dass das von diesem Sensor (4) entstammende Signal einer Analyse-Einheit (10) zugeführt wird, um den Umgebungseinfluss in Echtzeit zu kompensieren.

7. Verfahren nach Anspruch 6,
**dadurch gekennzeichnet, dass**
als Sensor (4) auf dem Brillengestell (1) vorgesehen sind: ein Lichtsensor (4) und/oder ein Beschleunigungssensor (4) und/oder ein Feuchtigkeitssensor (4).

8. Verfahren nach Anspruch 6 oder 7,
**dadurch gekennzeichnet, dass**
als Elektroden (2) trockene Elektroden (2) vorgesehen sind.

9. Verfahren nach einem der Ansprüche 6 bis 8,
**dadurch gekennzeichnet, dass**
ein Speichermodul (MMC) vorgesehen ist, um die kompensierten Signale zu speichern und mit Übertragungsmitteln (Bluetooth) die kompensierten Signale an ein externes Computersystem zu übertragen.
